# EUROPÄISCHE PATENTSCHRIFT

(11) **EP 2 038 391 B1**
(45) Veröffentlichungstag und Bekanntmachung des Hinweises auf die Patenterteilung: **18.04.2012**
(21) Anmeldenummer: 07730315.4
(22) Anmeldetag: 28.06.2007
(51) Int. Cl.: C11D 3/00, C11D 3/12

(54) **WASCH-, REINIGUNGS- UND PFLEGEMITTEL**
WASHING, CLEANING AND CARE PRODUCTS
AGENT DE LAVAGE, DE NETTOYAGE ET D'ENTRETIEN

(30) Priorität: 07.07.2006 DE 102006031897; 23.04.2007 DE 102007019428
(43) Veröffentlichungstag der Anmeldung: 25.03.2009
(73) Patentinhaber: Henkel AG & Co. KGaA, 40589 Düsseldorf (DE)
(72) Erfinder: MEINE, Georg, 40822 Mettmann (DE); RIEBE, Hans-Jürgen, 42697 Solingen (DE)
(86) Internationale Anmeldenummer: PCT/EP2007/056456
(87) Internationale Veröffentlichungsnummer: WO 2008/003631

(56) Entgegenhaltungen:
- EP-A1- 1 192 933
- WO-A-02/50224
- WO-A-96/23051
- WO-A-2005/108505
- US-A1- 2005 249 760
- US-A1- 2007 031 461

## Beschreibung

Die Erfindung betrifft ein Textilwasch- oder -pflegemittel, welches ein lichtaktives Bleichmittel enthält, das Titandioxid, welches modifiziert ist, umfasst. Ferner betrifft die Erfindung ein Verfahren zur Textilbehandlung unter Einsatz des lichtaktiven Bleichmittels.

Das Waschen, Reinigen und Pflegen der verschiedensten Objekte stellt ein menschliches Grundbedürfnis dar und die moderne Industrie versucht fortlaufend, diesen Bedürfnissen des Menschen in vielfältiger Weise gerecht zu werden, in dem sie eine unüberschaubare Vielzahl von Produkten aus dem Bereich der Wasch-, Reinigungs- und Pflegemittel zur Verfügung stellt.

Insbesondere farbige Anschmutzungen, auf Textilien stellen seit jeher eine besondere Herausforderung für die sinnvolle Konzeption von Wasch-, Pflege- und Reinigungsmitteln dar, weil solche farbige Anschmutzungen bereits in sehr geringer Menge zu deutlichen Flecken führen und als sehr störend wahrgenommen werden. Hinzu kommt, dass viele farbige Anschmutzungen äußerst fest auf der Textilfaser haften können. Aus diesen Gründen enthalten viele Wasch-, Pflege- oder Reinigungsmittel Bleichmittel.

Zu denjenigen Bleichmitteln des Standes der Technik, welche im Zusammenhang mit Wasch-, Pflege- oder Reinigungsmitteln die weiteste Verbreitung gefunden haben, zählen insbesondere die Bleichmittel auf Sauerstoffbasis wie z.B. Natriumperborat, Natriumpercarbonat sowie Wasserstoffperoxid, sowie Bleichmittel auf Chlorbasis, wie z.B. Natriumhypochlorit.

Mit diesen Bleichmitteln gehen jedoch Begrenzungen bei der Anwendung einher. Wasserstoffperoxid als solches weist nur eine geringe Lagerstabilität auf. Natriumperborat und Natriumpercarbonat zerfallen bekanntlich in Wasser unter Bildung von Wasserstoffperoxid. Dessen Bleichwirkung ist aber stark temperatur- und ph-abhängig. Unterhalb von 60°C ist die Bleichwirkung gering. Deshalb greift man oft auf Bleichaktivatoren wie N,N,N',N'-Tetraacetylethylendiamin (TAED) oder p-Nonanoyloxybenzolsulfonat (NOBS) zurück. In neutraler oder saurer Lösung besitzt Wasserstoffperoxid kein ausreichendes Bleichvermögen. Das Optimum der Bleichwirkung wird nur bei pH 10-11 erreicht.

Im Falle der Bleichmittel auf Chlorbasis, beispielsweise bei Natriumhypochlorit-Lösungen hat man Unverträglichkeitsprobleme mit anderen Waschmittelinhaltsstoffen, so dass Bleich- und Waschmittel oft getrennt dosiert werden müssen. Zur Stabilisierung der Bleichmittellösung ist im allgemeinen eine stark alkalische wässrige Lösung von Natriumhypochlorit erforderlich. Beim Ansäuern kommt es zur Freisetzung von gesundheitsschädlichem Chlorgas.

Hinzu kommt, dass die gängigen Bleichmittel wie Wasserstoffperoxid oder Natriumhypochlorit für viele Anwendungen, insbesondere Pflegeanwendungen, zu aggressiv sind.

Der Stand der Technik beschreibt den Einsatz von Fotokatalysatoren. So beschreibt WO 2005/108505 A1 einen speziellen kohlenstoffhaltigen Fotokatalysator auf Titandioxidbasis sowie ein Verfahren zu dessen Herstellung. Weiterhin beschriebt es diverse Verwendungen des Fotokatalysators, z.B. bei der Herstellung von Geweben. WO 02/50224 A1 beschreibt ein Halbleiterpulver, insbesondere mit Platin und/oder Rhodium dotiertes TiO₂, welches im sichtbaren Bereich aktiv ist. Ferner wird dort die Verwendung entsprechender wässriger Zusammensetzungen als Reinigungsmittel, beispielsweise als Küchen- oder Sanitärreiniger beschrieben. US 2005/0249760 A1 beschreibt Körperpflegemittel, welche einen Fotokatalysator auf Titandioxidbasis enthalten. EP 1 192 933 A1 beschreibt Zahnbleichungsmittel, welche einen Fotokatalysator auf Titandioxidbasis enthalten. WO 96/23051 A1 beschreibt den Einsatz von TiO₂ als bakterizides und fotoaktives Agens in einem Waschmittel.

Die Aufgabe der vorliegenden Erfindung lag deshalb darin, ein Wasch-, Pflege- oder Reinigungsmittel mit einem alternativen Bleichsystem zur Verfügung zu stellen.

Diese Aufgabe wurde überraschend durch den Gegenstand der Erfindung gelöst, nämlich durch die Bereitstellung eines Textilwasch- oder -pflegemittels, welches 0,01 bis 25 Gew.-% mit Kohlenstoff modifiziertes Titandioxid, bezogen auf das gesamte Mittel, als lichtaktives Bleichmittel umfasst, wobei das Mittel in einer lichtundurchlässigen Verpackung enthalten ist.

Das erfindungsgemäße lichtaktive Bleichmittel umfasst modifiziertes Titandioxid und kann vorteilhafterweise die vom menschlichen Auge wahrnehmbare Strahlung des sichtbaren Bereichs des Spektrums mit Wellenlängen zwischen 380 und 800 nm für die Zwecke der Fotobleiche ausnutzen und so eine allgemeine Reinigungswirkung entfalten, z.B. durch den Einfall von Tageslicht.

Die Lichtaktivität des lichtaktiven Bleichmittels bezieht sich vorteilhafterweise auf natürliches oder künstliches Licht mit einer Wellenlänge im Bereich von 10-1200 nm, vorzugsweise 300-1200 nm, insbesondere zwischen 380 und 800 nm.

Vorteilhafterweise reicht sogar das Licht, welches durch Glasfenster in geschlossene Wohnräume einfällt (diffuses Tageslicht) aus, um die angestrebte Reinigung (z.B. deutliche Verminderung farbiger Anschmutzungen) zu erreichen. Selbst Licht aus technischen Lichtquellen (Kunstlicht), wie z. B. aus handelsüblichen Glühlampen (Glühbirnen), Halogenlampen, Leuchtstoffröhren, Kompaktleuchtstofflampen (Energiesparlampen) sowie aus Lichtquellen auf Basis von Leuchtdioden, reicht aus, um die gewünschte Reinigung (z.B. Fleckentfernung) zu bewirken.

Ein großer Vorteil des erfindungsgemäßen Textilwasch- oder -pflegemittels liegt darin, dass es auch ohne den Einsatz von technischem Gerät, wie z.B. einer Waschmaschine, auskommt. Beispielsweise kann ein beflecktes Textil lokal mit dem erfindungsgemäßen Mittel behandelt werden, beispielsweise durch Einsprühen oder Tränken, und das behandelte Textil kann danach z.B. an der Leine aufgehängt werden, so dass durch Einwirkung von Licht eine Bleichwirkung bzw. allgemeine Reinigungswirkung erfolgt.

Ein weiterer Vorteil des erfindungsgemäßen Textilwasch- oder -pflegemittels liegt darin, dass es auch für die mobile Applikation geeignet ist. Z.B. kann ein Kaffeefleck oder Rotweinfleck mit einem flüssigen erfindungsgemäßen Mittel besprüht werden. Das beschmutzte Kleidungsstück muß dazu nicht einmal ausgezogen werden.

Das erfindungsgemäße Textilwasch- oder -pflegemittel entfaltet eine allgemeine Reinigungswirkung und leistet eine sehr gute Arbeit bei der Entfernung insbesondere farbiger Anschmutzungen mit Hilfe von Licht, insbesondere mithilfe von vom menschlichen Auge wahrnehmbarer Strahlung des sichtbaren Bereichs des Spektrums mit Wellenlängen zwischen 380 und 800 nm. Die behandelten Substrate werden dabei geschont. Vorteilhafterweise kann das erfindungsgemäße Textilwasch- oder -pflegemittel mit Hilfe von Licht im Wellenlängenbereich von 10-1200 nm, insbesondere auch mithilfe von UV-Strahlung (Wellenlänge 380-200 nm, vorzugsweise 380-320 nm) eine allgemeine Reinigungswirkung und vorzugsweise auch eine gute Arbeit bei der Entfernung farbiger Anschmutzungen leisten.

Das erfindungsgemäße Mittel ist besonders wirksam bei der Reduktion oder Entfernung von Flecken, die zurückgehen auf:
- rote bis blaue Anthocyanfarbstoffe, wie z.B. Cyanidin, z.B. aus Kirschen oder Heidelbeeren,
- rotes Betanidin aus der roten Beete,
- orangerote Carotinoide wie z.B. Lycopin, beta-Carotin, z.B. aus Tomaten oder Möhren,
- gelbe Curcumafarbstoffe, wie z.B. Curcumin, z.B. aus Curry und Senf,
- braune Gerbstoffe, z.B. aus Tee, Obst, Rotwein
- tiefbraune Huminsäure, z.B. aus Kaffee, Tee, Kakao,
- grünes Chlorophyll, z.B, aus grünen Gräsern,
- technische Farbstoffe aus Kosmetika, Tinten, Farbstiften

Ein weiterer Vorteil des erfindungsgemäßen Mittels besteht darin, dass auch Bakterien und andere Mikroorganismen abgetötet werden. Dies ist eine wichtiger Beitrag zu einer deutlichen Verminderung der Keimzahl und damit zu einer wirksamen Hygiene, z. B. Wäschehygiene.

Es wird so eine Keimverminderung auf ein gesundheitlich unbedenkliches Maß erreicht. Es ist bekannt, dass sich Bakterien und Pilze in feuchtem Milieu rasch vermehren. Bezogen z.B. auf Textilien bedeutet das, dass nur dann, wenn (gewaschene) Wäsche trocken gelagert wird, es nicht zu einer Vermehrung von Mikroorganismen kommt. Dennoch können diese über mehrere Wochen auf trockener Wäsche überleben. In feuchtem Milieu kommt es dagegen zu einer starken Vermehrung der Mikroorganismen auf der Wäsche.

Durch das heute übliche Textilwaschen bei möglichst tiefen Temperaturen (30°C und 40°C) kann es vorkommen, dass die Keimreduzierung auf der Wäsche selbst nach der Maschinenwäsche nur suboptimal ist. Beim nachfolgenden Trocknen an der Luft ist im Gegensatz zum Trocknen im Wäschetrockner nicht mit einer weiteren Verminderung der Keime zu rechnen. Im Gegenteil kann es zu einem Bakterienwachstum kommen, insbesondere beim langsamen Lufttrocknen, wie es in der heutigen, städtisch geprägten Zivilisation üblich ist, da dort mangels Platz oder Zugriff auf Freiluftflächen nur in schlecht belüftbaren Räumen oder in abgeschlossenen Räumen getrocknet werden kann. Insbesondere für stark belastete Kleidungsstücke (z.B. Strümpfe, Unterhosen, Geschirrtücher) läßt sich dann keine ausreichende hygienische Reinheit mehr garantieren. Besonders hier hilft das erfindungsgemäße Mittel.

Bei der Behandlung von Textilien, mit dem erfindungsgemäßen Mittel wird vorteilhafterweise eine wirksame Menge des modifizierten Titanoxids auf die Textiloberfläche aufgebracht. Dieses sorgt dann für eine angemessene Keimverminderung sowie Reduktion farblicher Anschmutzungen.

Eine wirksame Menge des modifizierten Titanoxids liegt bereits dann vor, wenn zumindest eine graduelle Reinigungswirkung (z.b. Bleichwirkung) erzielt wird. Dies läßt sich leicht im Routineversuch klären. Die Reinigungswirkung kann sich z.B. in Abhängigkeit von der Lichststrahlung oder der Verschmutzungsart oder-intensität z. B. auch über mehrere Stunden hinziehen.

Ein weiterer Vorteil des erfindungsgemäßen Mittels liegt darin, daß es neben der vorteilhafterweise auftretenden allgemeinen Reinigungswirkung und neben der vorteilhafterweise auftretenden Hygienewirkung vorzugsweise auch vorteilhaft bei der Verminderung, Beseitigung oder Neutralisierung fötider Gerüche eingesetzt werden kann. Der fötide Geruch kann dabei vorteilhafterweise so gemindert werden, daß eine Geruchsbelästigung nicht mehr vorliegt.

Riechstoffe, wie vorzugsweise etherische Öle, können in allen erfindungsgemäßen Mitteln enthalten sein.

Die Konfektionierung der erfindungsgemäßen Mittel richtet sich vorzugsweise nach den Bedürfnissen des Verwendungszweckes.

Erfindungsgemäße Mittel können in fester, halbfester, flüssiger, disperser, emulgierter, suspendierter, aerosoler oder gelförmiger Form vorliegen. Feste und flüssige Mittel können z.B. in Säckchen oder (vorzugsweise selbstauflösenden) Portionsbeuteln (pouches) abgepackt sein, insbesondere auch in Mehrkammerpouches. Unter den Begriff der Flüssigkeit fallen im Sinne der Erfindung auch jegliche Festkörperdispersionen in Flüssigkeiten. Erfindungsgemäße Mittel können auch als Pasten, Salben, Lotionen oder Cremes vorliegen. Feste Mittel können als rieselfähige Pulver, als Schuppen, als feste Blöcke, als Stücke (z.B. Seifenstücke), als Kugeln oder Sticks oder Tabletten, insbesondere mehrlagige Tabletten vorliegen.

Das erfindungsgemäße Mittel ist in einem lichtundurchlässigen Behälter bzw. einer lichtundurchlässigen Verpackung enthalten.

Die lichtundurchlässige Verpackung ermöglicht eine verbesserte Lagerstabilität des erfindungsgemäßen Textilwasch- oder -pflegemittels.

Insbesondere solche Textilwasch- oder -pflegemittel, die nicht in fester, sondern in flüssiger, disperser, emulgierter, suspendierter, aerosoler oder gelförmiger Form vorliegen, profitieren von einer lichtundurchlässigen Verpackung. Aber auch feste und halbfeste Textilwasch- oder - pflegemittel profitieren von einer lichtundurchlässigen Verpackung.

Wenn das erfindungsgemäße Textilwasch- oder -pflegemittel also in fester, halbfester, flüssiger, disperser, emulgierter, suspendierter, aerosoler oder gelförmiger Form in einer lichtundurchlässigen Verpackung vorliegt, so entspricht dies einer bevorzugten Ausführungsform.

Insbesondere flüssige Waschmittel und flüssige Textilbehandlungsmittel, wie z.B. Weichspüler oder Hygienespüler profitieren von einer lichtundurchlässigen Verpackung.

Die Bereitstellung lichtundurchlässiger Verpackungen bereitet dem Fachmann keine Probleme. Besonders bevorzugte Verpackungsmaterialien sind insbesondere Polyethylen und Polypropylen. Wenn das Material der lichtundurchlässigen Verpackung daher Polyethylen und/oder Polypropylen umfaßt, so liegt eine bevorzugte Ausführungsform vor. Die Verpackungsmaterialien können auch eingefärbt und/oder beschichtet sein.

Insbesondere ist es vorteilhaft, wenn die Verpackung undurchlässig für elektromagnetische Strahlung im Wellenlängenbereich von 10-1200 nm, vorzugsweise 380 - 800 nm ist. Dies entspricht einer bevorzugten Ausführungsform.

"Lichtundurchlässig" bzw. "undurchlässig" bedeutet, dass die Transmission, also die Durchlässigkeit der Verpackung für Licht, insbesondere für elektromagnetische Strahlung im Wellenlängenbereich von 10-1200 nm, vorzugsweise 380 - 800 nm, um mindestens 50%, 60%, 70%, 80%, 90%, 95% oder sogar 99% reduziert ist, im Vergleich zu einem barrierefreien Zustand, also völliger Lichtdurchlässigkeit. Am vorteilhaftesten ist es, wenn die Lichtundurchlässigkeit der Verpackung absolut ist. Die Transmission kann nach üblichen spektroskopischen oder photometrischen Methoden bestimmt werden unter Einsatz üblicher Spektrometer oder Photometer.

In einer besonderen Ausführungsform liegen die erfindungsgemäßen Mittel in flüssiger Form vor. Die Applikation kann dabei vorzugsweise mit Sprühvorrichtungen erfolgen. Diese Sprühvorrichtungen enthalten in einem Behälter eine Füllung aus dem erfindungsgemäßen (flüssigen, breiartigen oder pulverförmigen) Textilwasch- oder -pflegemittel. Die Füllung kann unter dem Druck eines Treibmittels stehen (Druckgasdosen, Druckgaspackungen, Aerosolpackungen) oder es kann sich um einen mechanisch zu bedienenden Pumpzerstäuber (Pumpsprays) handeln. Die Behälter weisen eine Entnahmevorrichtung auf, vorzugsweise in Gestalt von Ventilen, die die Entnahme des Inhalts als Nebel, Rauch, Schaum, Pulver, Paste oder Flüssigkeitsstrahl ermöglichen. Als Behälter für die Sprühvorrichtungen kommen vor allem zylindrische Gefäße aus Metall (Aluminium, Weißblech, Rauminhalt vorzugsweise <1000 mL), geschütztem bzw. nichtsplitterndem Glas oder Kunststoff (Rauminhalt vorzugsweise <220 mL) bzw. splitterndem Glas oder Kunststoff (Rauminhalt vorzugsweise <150 mL) in Frage.

Die Applikation kann z.B. im Falle flüssiger Mittel vorzugsweise auch mit einem Roller-Applikator erfolgen, wie er z.B. aus dem Bereich der Deo-Roller bekannt ist. Solche Roller weisen eine in einem Kugelbett gelagerte Kugel auf, welche durch Bewegung über eine Oberfläche bewegt werden kann. Dabei nimmt die Kugel etwas von dem zu verteilenden Mittel auf und befördert dieses an die zu behandelnde Oberfläche.

Die Applikation kann z.B. auch mit sogenannten Feuchttüchern, d.h. für den Anwender vorgefertigten, vorzugsweise einzeln abgepackten, Feuchttüchern erfolgen, wie sie z.B. aus dem Bereich der feuchten Toilettenpapiere wohlbekannt sind. Solche Feuchttücher, welche vorteilhafterweise auch Konservierungsstoffe enthalten können, sind dann vorteilhafterweise mit einem erfindungsgemäßen Mittel imprägniert oder beaufschlagt, vorteilhafterweise sind sie einzeln verpackt. Sie können dann z.B. als mobiles Fleckentfernungsmittel eingesetzt werden. Wenn ein Verbraucher infolge eines unglücklichen Geschicks z. B. einen Rotweinfleck auf sein Oberhemd macht, so kann er ein solches Feuchtuch hernehmen und durch Reiben des Feuchttuches an dem befleckten Textil den Fleck vorteilhafterweise entfernen oder zumindest den Makel mindern.

Die erfindungsgemäßen Mittel, vorzugsweise flüssigen Mittel, können auch mehrphasig sein, die Phasen können z.B. horizontal angeordnet sein, also übereinander, oder vertikal, also nebeneinander, angeordnet sein. Es kann sich auch um ein disperses System handeln, bei dem z.B. die festen Bestandteile inhomogen in der flüssigen Matrix verteilt sind, so daß ein solches disperses System vor der Anwendung geschüttelt werden sollte.

Bevorzugte Waschmittel im Sinne der Erfindung sind u.a. die sogenannten Waschhilfsmittel für Textilien. Dazu zählen insbesondere:
(a) Schwerpunktverstärker wie z.B. Feltentferner. Diese dienen vorwiegend zur (Vor)-Behandlung lokal auftretender Fett- und/oder Pigmentverschmutzungen, und werden direkt auf die Textilien aufgetragen.
(b) Einweichmittel. Diese sind zumeist stärker alkalisch eingestellte Hilfsmittel (bis etwa pH 13) mit geringerem Tensidanteil. Sie begünstigen Quellungsvorgänge bei Anschmutzungen und erniedrigen die Schmutzhaftung.
(c) separate Bleichmittel, wie Fleckensalze, flüssige Bleichen, Waschkraftverstärker. Sie können sowohl als Waschmittel-Additiv wie auch zur Vorbehandlung verwendet werden.
(d) separate Enthärter, vorzugsweise enthaltend Builder, wie z.B. Citrate und/oder Zeolithe, und Dispergiermittel, wie z.B. Polycarboxylate.
(e) separate Verfärbungsinhibitoren,
(f) Spezialfleckentfernungsmittel für einzelne Fleckenarten
(g) Textilvorbehandlungsmittel, vorzugsweise flüssig oder pastös, vorzugsweise Spray, zur gezielten Fleckenvorbehandlung.

Bevorzugte Mittel im Sinne der Erfindung sind auch Nachbehandlungsmittel für Textilien. Dazu zählen insbesondere:
(a) Weichspüler, auch Avivagemittel oder Gewebeconditioner genannt, sowie entsprechende Tücher für die Trocknerapplikation
(b) Wäschesteifen, welche die Wirkung haben, der Wäsche eine steife und füllige Formgebung zu verleihen
(c) Formspüler, welche die Wirkung haben, dass damit behandelte Textilien im Faserverband gefestigt werden, so dass die Wäsche einen leichten Appretur- bis kräftigen Stärkegriff erhält
(d) Hygienespüler, welche vorzugsweise dem letzten Spülgang zugesetzt werden und einen antimikrobiellen Wirkstoff sowie vorzugsweise nichtionische Tenside enthalten
(e) Bügelhilfen, welche beispielsweise aufgesprüht werden und auch ohne Bügeln in gewissem Ausmaß faserglättend wirken, wenn z.B. nach dem Aufsprühen die noch feuchte Wäsche mit der Hand gestrafft wird
(f) (Gardinen)-Weißspüler, welche den Zweck haben, den Weißgrad (der Gardinen) zu erhöhen und ggf. faserfestigend wirken
(g) Pflegespüler, welche das Textil bei der Textilbehandlung (z.B. Maschinenwäsche) mit Wirkstoffen ausstatten (z.B. Öle), welche beim Tragen des Textils an die menschliche Haut abgegeben werden können und der Haut zum Vorteil gereichen bzw. sie pflegen
(h) Bügelwasser, welche vorteilhafterweise mineralarmes oder vollentsalztes Wasser, vorzugsweise Konservierungsmittel und Duftstoffe umfassen, zur Verwendung in Dampfbügeleisen
(i) Textilerfrischer, d.h. Produkte welche Gerüche aus vielen textilen Materialien entfernen, z.B. via Einkapselung der zu entfernenden Gerüche mit Hilfe geeignet Mittel wie z.B. Cyclodextrinen oder aber via anderer Wirkstoffe, wie z.B. Zink-Ricinoleat.
(j) flüssiges Nachbehandlungsmittel, vorzugsweise Spray, zur gezielten Flecken(nach)behandlung

Besonders bevorzugt sind ganz allgemein jegliche Textilbehandlungsmittel, wie beispielsweise Waschmittel oder Weichspüler, in flüssiger wie in fester Form. Bevorzugte Mittel im Sinne der Erfindung sind daher Waschmittel. Dazu zählen insbesondere:
(a) Vollwaschmittel, (enthält vorzugsweise. Bleichmittel, optische Aufheller, Enzyme usw.)
(b) Colorwaschmittel, (vorzugsweise enthaltend Verfärbungsinhibitoren, Cellulasen usw.)
(c) Feinwaschmittel, (vorzugsweise geringere Alkalität),
(d) Spezialwaschmittel, wie z.B. insbesondere
   i) Wollwaschmittel, (vorzugsweise pH-neutral)
   ii) Gardinenwaschmittel,
   iii) Waschmittel für die Handwäsche,
   iv) Waschmittel mit Zusatznutzen, wie vorzugsweise
      - Waschmittel mit Geruchsabsorber
      - UV-Schutz-Waschmittel,
      - Hygiene-Waschmittel,
      - Bügelerleichterungswaschmittel,
      - Spezialwaschmittel für schwarze oder weiße Wäsche,
      - Sensitiv-Waschmittel, vorzugsweise enthaltend pflegende Substanzen, wie z.B. Mandelöl, Aloe Vera Extrakt usw.
      - Duftintensiv- bzw. Aroma-Waschmittel)

Das mit Kohlenstoff modifizierte Titandioxid ist in dem erfindungsgemäßen Mittel in Mengen von vorzugsweise 0,01 bis 5 Gew.-% enthalten, bezogen auf das gesamte Mittel. Die Obergrenze für das modifizierte Titandioxid kann auch bei 20 Gew.-%, 15 Gew.-%, 10 Gew.-%, 5 Gew.-%, 1 Gew.-%, 0,5 Gew.-%, 0,1 Gew.-%, 0,05 Gew.-% liegen, bezogen auf das gesamte Mittel.

Das im erfindungsgemäßen Mittel enthaltene modifzierte Titandioxid kann vorteilhafterweise eine Teilchengröße im Bereich zwischen 2 bis 600 nm aufweisen, also z.B. 3 bis 150 nm oder z.B. 4 bis 100 nm oder z.B. 5 bis 75 nm oder z.B. 10 bis 30 nm oder z.B. 200 bis 400 nm. Die Teilchengröße des modifizierten Titandioxids kann zwar vorzugsweise im Bereich von von 100-500 nm, vorteilhafterweise 200-400 nm liegen. Es kann auch bevorzugt sein, dass die Teilchengröße sehr klein ist, z.B. im Bereich von 2-150 nm, vorzugsweise 3-100 nm, vorteilhafterweise 4-80 nm oder z.B. 5-50 nm oder z.B. 8-30 nm oder z.B. 10-20 nm liegt. Die Teilchengröße kann z.B. vorteilhafterweise bei Werten wie 5 nm, 10 nm, 15 nm, 20 nm, 25 nm, 30 nm, 35 nm, 40 nm, 45 nm, 50 nm oder 60 nm liegen. Insbesondere sehr kleine Teilchengrößen unter 50 nm, unter 40 nm, unter 30 nm oder unter 20 nm können bevorzugt sein.

Es kann vorteilhaft sein, bei der Herstellung des modifizierten Titandioxids von mikronisiertem Titandioxid auszugehen, also von Titandioxid mit sehr geringer Teilchengröße, z.B. zwischen 2 und 150 nm oder z.b. zwischen 5 und 100 nm. Die Teilchengröße kann dann z.B. vorteilhafterweise bei Werten wie 5 nm, 10 nm, 15 nm, 20 nm, 25 nm, 30 nm, 35 nm, 40 nm, 45 nm, 50 nm oder 60 nm liegen. Solche Werte sind bevorzugt.

Die spezifische Oberfläche nach BET liegt für das modifizierte Titandioxid vorteilhafterweise bei 50-500 m²/g, vorzugsweise 100 bis 400 m²/g, in weiter vorteilhafter Weise 200 bis 350 m²/g, insbesondere 250 bis 300 m²/g.

Die Schüttdichte des modifizierten Titandioxids liegt vorzugsweise im Bereich von 100 bis 800 g/l, vorteilhafterweise von 200 bis 600 g/l, insbesondere von 300-500 g/l. Beispielsweise kann die Schüttdichte 350 g/l, 400 g/l oder 500 g/l betragen.

Der Kohlenstoffgehalt des mit Kohlenstoff modifizierten Titandioxids kann nach einer bevorzugten Ausführungsform im Bereich von 0,01 bis 10 Gew.-% vorzugsweise von 0,05 bis 5,0 Gew.-%, vorteilhafterweise von 0,3 bis 1,5 Gew.%, insbesondere von 0,4 bis 0,8 Gew.% liegen. Vorteilhafterweise liegt der TiO₂-Gehalt des mit Kohlenstoff modifizierten Titandioxids z.B. über 95 Gew.-%, 96 Gew.-%, 97 Gew.-%, 98 Gew.-% oder 99 Gew.-%, bezogen auf das gesamte mit Kohlenstoff modifizierte Titandioxid.

Wenn der Kohlenstoff nur in einer Oberflächenschicht der Titandioxid-Partikel eingelagert ist, so liegt eine bevorzugte Ausführungsform vor. Das modifizierte Titandioxid kann vorteilhafterweise zusätzlich Stickstoff enthalten.

Wenn die spezifische Oberfläche des modifizierten Titandioxids, nach BET (BET vorteilhafterweise nach DIN ISO 9277: 2003-05 bestimmt, vorzugsweise vereinfacht auch nach DIN 66132: 1975-07) vorzugsweise 50 bis 500 m²/g, vorteilhafterweise 100 bis 400 m²/g, in weiter vorteilhafter Weise 200 bis 350 m²/g, insbesondere 250 bis 300 m²/g beträgt, so liegt ebenfalls eine bevorzugte Ausführungsform vor.

Das mit Kohlenstoff modifizierte Titandioxid kann nach einer bevorzugten Ausführungsform z.B. dadurch erhalten werden, dass man eine Titanverbindung, welche eine spezifische Oberfläche von vorzugsweise mindestens 50 m²/g nach BET aufweist, mit einer organischen Kohlenstoffverbindung innig vermischt und die Mischung bei einer Temperatur von bis zu 350°C thermisch behandelt wird.

Die dabei einsetzbare kohlenstoffhaltige Substanz kann nach einer bevorzugten Ausführungsform eine Kohlenstoffverbindung sein, welche zumindest eine funktionell Gruppe enthält, vorzugsweise ausgewählt aus OH, CHO, COOH, NHx, SHx. Insbesondere kann es sich bei der Kohlenstoffverbindung um eine Verbindung aus der Gruppe Ethylenglykol, Glycerin, Bernsteinsäure, Pentaerythrit, Kohlehydrate, Zucker, Stärke, Alkylpolyglucoside, Organoammoniumhydroxide oder Mischungen davon handeln. Es ist auch möglich, dass als kohlenstoffhaltige Substanz Ruß oder Aktivkohle eingesetzt wird.

Es kann auch bevorzugt sein, dass die kohlenstoffhaltige Substanz, welche mit der Titanverbindung vorteilhafterweise gemischt wird, um nach der thermischen Behandlung zu dem modifizierten Titandioxid zu gelangen, eine Zersetzungstemperatur von höchstens 400°C bevorzugt < 350°C und insbesondere bevorzugt < 300°C aufweist.

Die zur Herstellung des modifizierten Titandioxids vorzugsweise einsetzbare Titanverbindung, welche gemäß zuvor genannter bevorzugter Ausführungsform mit einer organischen Kohlenstoffverbindung innig vermischt wird, kann ein amorphes, teilkristallines oder kristallines Titanoxid bzw. wasserhaltiges Titanoxid oder ein Titanhydrat oder ein Titanoxyhydrat sein, was wiederum einer bevorzugten Ausführungsform entspricht.

Die thermische Behandlung der Mischung aus der Titanverbindung und der Kohlenstoffverbindung kann nach einer bevorzugten Ausführungsform vorteilhafterweise in einem kontinuierlich zu betreibenden Calcinieraggregat, vorzugsweise einem Drehrohrofen durchgeführt werden.

Das modifizierte Titandioxid läßt sich, insbesondere im Kontext des zuvor Beschriebenen, vorzugsweise z.B. dadurch erhalten, dass man ein Titandioxid (z.B. mit einer Teilchengröße im Bereich zwischen 2 bis 600 nm oder z.B. 3 bis 150 nm oder oder z.B. 4 bis 100 nm oder z.B. 5 bis 75 nm oder z.B. 10 bis 30 nm oder z.B. 200 bis 400 nm ), wie etwa handelsüblich erhältlich in Pulver-oder Schlammform, hernimmt und aus diesem eine Suspension in einer Flüssigkeit, wie vorzugsweise Wasser, herstellt. Zu der Suspension wird dann vorteilhafterweise eine kohlenstoffhaltige Substanz zugegeben und es wird gemischt. Das Mischen kann unterstützt werden durch den Einsatz von Ultraschall. Der Mischvorgang (z.B. Rühren) kann vorzugsweise mehrere Stunden andauern, vorzugsweise 2, 4, 6, 8, 10 oder 12 Stunden oder sogar länger.

Bezogen auf die Feststoffe der Suspension beträgt die Menge der Kohlenstoffverbindung vorteilhafterweise 1-40 Gew.-%, dementsprechend die Menge der Titanverbindung vorzugsweise 60-99 Gew.-%.

Danach wird die Flüssigkeit entfernt, beispielsweise durch Filtration, Abdampfen im Vakuum oder Dekantieren, und der Rückstand wird vorzugsweise getrocknet (z.B. vorzugsweise bei Temperaturen von 70-200°C, vorteilhafterweise über mehrere Stunden, beispielsweise mindestens 12 Stunden)) und anschließend calziniert, beispielsweise bei einer Temperatur von mindestens 260°C, vorzugsweise z.B. bei 300°C, vorzugsweise über einen Zeitraum von mehreren Stunden, vorzugsweise 1-4 Stunden, insbesondere 3 Stunden. Die Calcinierung kann vorteilhafterweise in einem geschlossenen Gefäß stattfinden.

Es kann vorteilhaft sein, daß die Calcinierungstemperatur, z.B. 300°C, innerhalb einer Stunde erreicht wird (langsames Aufheizen auf 300°C).

Dabei ist vorzugsweise ein Farbwechsel des Pulvers von weiß über dunkelbraun nach beige bzw. leicht gelb-bräunlich festzustellen. Zu langes Erhitzen führt zu inaktiven, farblosen Pulvern. Der Fachmann kann dies mit wenigen Routineversuchen abschätzen. Die Calcinierung kann z.B. vorteilhafterweise so lange erfolgen, bis nach einem Farbwechsel des Pulvers von weiß über dunkelbraun ein weiterer Farbwechsel auf beige bzw. leicht gelb-bräunlich statfindet.

Eine maximale Temperatur von 350°C sollte dabei vorzugsweise nicht überschritten werden. Bei der thermischen Behandlung kommt es zu einer Zersetzung der organischen Kohlenstoffverbindung an der Oberfläche der Titanverbindung, so dass vorzugsweise ein modifiziertes Titandioxid entsteht, das vorzugsweise 0,005-4 Gew.-% Kohlenstoff enthält.

Nach der thermischen Behandlung wird das Produkt mit bekannten Verfahren vorteilhafterweise deagglomeriert, beispielsweise in einer Stiftmühle, Strahlmühle oder Gegenstrahlmühle. Die zu erzielende Kornfeinheit hängt von der Korngöße der Ausgangstitanverbindung ab. Die Kornfeinheit oder spezifische Oberfläche des Produkts liegt nur geringfügig niedriger, aber in der gleichen Größenordnung wie die des Edukts. Die angestrebte Kornfeinheit des Photokatalysators hängt von dem Einsatzbereich des Photokatalysators ab. Sie liegt üblicherweise im Bereich wie bei TiO₂-Pigmenten, kann aber auch darunter oder darüber liegen.

Die Teilchengröße des modifizierten Titandioxid kann vorzugsweise im Bereich von 2-600 nm liegen, vorzugsweise im Bereich von 100-500 nm, vorteilhafterweise 200-400 nm. Es kann auch bevorzugt sein, dass die Teilchengröße sehr klein ist, z.B. im Bereich von 2-150 nm, vorzugsweise 3-100 nm, vorteilhafterweise 4-80 nm oder z.B. 5-50 nm oder z.B. 8-30 nm oder z.B. 10-20 nm liegt. Es kann vorteilhaft sein, bei der Herstellung des modifizierten Titandioxids von mikronisiertem Titandioxid auszugehen, also von Titandioxid mit sehr geringer Teilchengröße, z.B. zwischen 2 und 150 nm oder z.b. zwischen 5 und 100 nm. Die Teilchengröße kann dann z.B. vorteilhafterweise bei Werten wie 5 nm, 10 nm, 15 nm, 20 nm, 25 nm, 30 nm, 35 nm, 40 nm, 45 nm, 50 nm oder 60 nm liegen. Solche Werte sind bevorzugt.

Die spezifische Oberfläche nach BET liegt für das modifizierte Titandioxid vorteilhafterweise bei 50-500 m²/g, vorzugsweise 100 bis 400 m²/g, in weiter vorteilhafter Weise 200 bis 350 m²/g, insbesondere 250 bis 300 m²/g.

Die Schüttdichte des vorzugsweise modifizierten Titandioxids liegt vorzugsweise im Bereich von 100 bis 800 g/l, vortielhafterweise von 200 bis 600 g/l, insbesondere von 300-500 g/l. Beispielsweise kann die Schüttdichte 350 g/l, 400 g/l oder 500 g/l betragen.

Nach einer bevorzugten Ausführungsform liegt das modifizierte Titandioxid in der Kristallmodifikation Anatas vor.

In einer bevorzugten Ausführungsform der Erfindung können die erfindungsgemäßen Mittel auch Cyclodextrine, Polyvinylamine und/oder Polyethylenimine enthalten, vorzugsweise aber Cyclodextrin-TiO₂-Komplexe, insbesondere auf Basis des modifizierten TiO₂, wie es zuvor beschrieben wurde.

Einen weiteren Gegenstand der Erfindung bildet ein Verfahren zur Behandlung eines Textils, umfassend die Kontaktierung des Textils mit einem erfindungsgemäßen Textilwasch- oder -pflegemittel, bei und/oder gefolgt von einer Exponierung der Oberfläche des behandelten Stoffes an Licht mit einer Wellenlänge im Bereich von 300-1200 nm, vorzugsweise 400-800 nm. Für eine bevorzugte Entfaltung der Wirksamkeit des Fotobleichmittels ist die Anwesenheit von vorzugsweise Sauerstoff und/oder Wasser (z.B. aus Luft, also Luftfeuchte) erforderlich. Dazu genügt z.B. der in Wasser anwesende, gelöste Sauerstoff oder der in der Feuchtigkeit gelöste Sauerstoff oder Luftsauerstoff. Die Belichtung kann auch in einem Behandlungsbad erfolgen. Für das genannte Verfahren ist Licht im Wellenlängenbereich von 10-1200 nm nutzbar.

Dieses Verfahren bietet die Vorteile der Photodesinfektion, eines guten Farb- und Textilschutzes, einer allgemeinen Substratschonung. Außerdem wird die Entfernung von Flecken aus dem behandelten Gewebe erheblich verbessert. Zur Klarstellung sein angemerkt, dass das in dem erfindungsgemäßen Verfahren einsetzbare flüssige Textilwasch- oder -pflegemittel durchaus nur aus den Komponenten modifiziertes Titandioxid und Lösungsmittel, vorzugsweise umfassend Wasser, bestehen können. Vorteilhaftweise sind in solchen flüssigen Wasch-, Pflege- oder Reinigungsmitteln aber auch Tenside und ggf. weitere, optionale Inhaltsstoffe enthalten. Mögliche Inhaltsstoffe, die in dem Verfahren einsetzbar sind, werden weiter unten beschrieben.

Zur Klarstellung sei hier angemerkt, dass ein in dem erfindungsgemäßen Verfahren einsetzbares flüssiges Wasch-, Pflege- oder Reinigungsmittel durchaus nur aus dem Komponenten modifiziertes Titandioxid und Lösungsmittel bestehen kann.

Die erfindungsgemäßen Textilwasch- oder -pflegemittel können neben dem modifizierten Titandioxid weitere Inhaltsstoffe enthalten. Es sei angemerkt, dass unter den Begriff der Pflegemittel auch die Konditioniermittel fallen.

Nach einer bevorzugten Ausführungsform weist das erfindungsgemäße Textilwasch- oder -pflegemittel neben dem modifizierten Titandioxid wenigstens einen weiteren, vorzugsweise mehrere Inhaltsstoffe auf, insbesondere wasch-, pflege- und/oder reinigungsaktive Inhaltsstoffe, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositionsmittel, Antistatika, Buildersubstanzen, Bleichmittel, Bleichaktivatoren, Bleichstabilisatoren, Bleichkatalysatoren, Bügelhilfsmittel, Cobuilder, Dispergiermittel, Einlaufverhinderer, Elektrolyte, Enzyme, Farbschutzstoffe, Färbemittel, Farbstoffe, Farbübertragungsinhibitoren, Fluoreszensmittel, Fungizide, Germizide, geruchskomplexierende Substanzen, Hilfsmittel, Hydrotrope, Klarspüler, Komplexbildner, Konservierungsmittel, Korrosionsinhibitoren, optische Aufheller, Parfümträger, Perlglanzgeber, pH-Stellmittel, Phobier-und Imprägniermittel, Polymere, Riechstoff(e) (Parfüm(öl)), Quell- und Schiebefestmittel, Schauminhibitoren, Schichtsilikate, schmutzabweisende Stoffe, Silberschutzmittel, Silikonöle, UV-Schutz-Substanzen, Viskositätsregulatoren, Verdickungs-mittel, Verfärbungsinhibitoren, Vergrauungsinhibitoren, Vitamine und/oder Weichspüler.

Im Folgenden werden bevorzugte Inhaltsstoffe der erfndungsgemäßen Mittel näher beschrieben. Anionische Tenside sind bevorzugt in den erfindungsgemäßen Mitteln enthalten. Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), z.B. die α-sulfonierten Methylester der hydrierten Kokos-, Palmkern- oder Talgfettsäuren geeignet.

Der Gehalt des erfindungsgemäßen Mittels an anionischen Tensiden, vorzugsweise an den genannten anionischen Tensiden, kann in weiten Bereichen variieren, je nachdem welchem Zweck das betreffende Mittel dient. So kann ein erfindungsgemäßes Mittel sehr große Mengen Aniontensid enthalten, vorzugsweise bis zu einer Größenordnung von bis zu 40, 50 oder 60 Gew.- oder mehr. Ebenso kann ein erfindungsgemäßes Mittel nur sehr geringe Mengen Aniontensid enthalten, beispielsweise weniger als 15 oder 10 Gew.-% oder weniger als 5 Gew.-% oder noch weniger. Vorteilhafterweise sind in den erfindungsgemäßen Mitteln jedoch Aniontenside in Mengen von 0,1 bis 40 Gew.-% und insbesondere 5 bis 30 Gew.-% enthalten, wobei Konzentrationen oberhalb von 10 Gew.-% und sogar oberhalb von 15 Gew.-% besondere Bevorzugung finden. Nach einer bevorzugten Ausführungsform enthält das erfindungsgemäße Mittel anionische Tenside, vorzugsweise in Mengen von zumindest 0,01 Gew.-%, bezogen auf das gesamte Mittel. Nach einer anderen bevorzugten Ausführungsform kann das erfindungsgemäße Mittel frei von Aniontensid sein.

Zusätzlich zu den genannten anionischen Tensiden, aber auch unabhängig von diesen, können in den erfindungsgemäßen Mitteln Seifen enthalten sein. Geeignet sind insbesondere gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische. Der Gehalt des Mittels an Seifen beträgt, unabhängig von anderen Aniontensiden, vorzugsweise nicht mehr als 3 Gew.-% und insbesondere 0,5 bis 2,5 Gew.-%, bezogen auf das gesamte Mittel. Nach einer anderen bevorzugten Ausführungsform ist das erfindungsgemäße Mittel von Seife.

Die anionischen Tenside und Seifen können in Form ihrer Natrium-, Kalium- oder Ammoniumsalze sowie als lösliche Salze organischer Basen, wie Mono-, Di- oder Triethanol-amin, vorliegen. Vorzugsweise liegen sie in Form ihrer Natrium- oder Kaliumsalze, insbesondere in Form der Natriumsalze vor. Anionische Tenside und Seifen können auch in situ hergestellt werden, indem in die sprühzutrocknende Zusammensetzung die Aniontensidsäuren und gegebenenfalls Fettsäuren eingebracht werden, welche dann durch die Alkaliträger in der sprühzutrocknenden Zusammensetzung neutralisiert werden.

Vorteilhafterweise können nichtionische Tenside in den erfindungsgemäßen Mitteln ebenfalls enthalten sein, sowohl in festen wie in flüssigen Mitteln. Beispielsweise kann ihr Gehalt bis zu 2 oder 3 oder 5 Gew.-% betragen. Es können auch größere Mengen an nichtionischem Tensid enthalten sein, beispielsweise bis zu 5 Gew.-% oder 10 Gew.-% oder 15 Gew.-% oder 20 Gew.-%, 30 Gew.-%, 40 Gew.-%, 50 Gew.-% oder sogar darüber hinaus, falls es zweckmäßig ist. Sinnvolle Untergrenzen können bei Werten von 0,01, 0,1, 1, 2, 3 oder 4 Gew.-% liegen.

Vorzugsweise sind die nichtionischen Tenside aber in größeren Mengen, also bis zu 50 Gew.-%, vorteilhafterweise von 0,1 bis 40 Gew.-%, besonders bevorzugt von 0,5 bis 30 und insbesondere von 2 bis 25 Gew.-%, jeweils bezogen auf das gesamte Mittel, enthalten. Nach einer bevorzugten Ausführungsform enthält das erfindungsgemäße Mittel nichtionische Tenside, vorzugsweise in Mengen von zumindest 0,1 Gew.-%, bezogen auf das gesamte Mittel. Nach einer anderen bevorzugten Ausführungsform kann das erfindungsgemäße Mittel frei von Niotensid sein.

Vorteilhafterweise können alle aus dem Stand der Technik bekannten nichtionischen Tenside in den erfindungsgemäßen Mitteln enthalten sein.

Die erfindungsgemäßen Textilwasch- oder -pflegemittel können vorzugsweise auch kationische Tenside enthalten. Geeignete Kationtenside sind beispielsweise oberflächenaktive quaternäre Verbindungen, insbesondere mit einer Ammonium-, Sulfonium-, Phosphonium-, Jodonium- oder Arsoniumgruppe. Durch den Einsatz von quaternären oberflächenaktiven Verbindungen mit antimikrobieller Wirkung kann das Mittel mit einer antimikrobiellen Wirkung ausgestaltet werden bzw. dessen gegebenenfalls aufgrund anderer Inhaltsstoffe bereits vorhandene antimikrobielle Wirkung verbessert werden.

Die erfindungsgemäßen Textilwasch- oder -pflegemittel können ein oder mehrere kationische Tenside enthalten, vorteilhafterweise in Mengen, bezogen auf die Gesamtzusammensetzung, von 0 bis 30 Gew.-%, noch vorteilhafter größer 0 bis 20 Gew.-%, vorzugsweise 0,01 bis 10 Gew.-%, insbesondere 0,1 bis 5 Gew.-%. Geeignete Mindestwerte können auch bei 0,5, 1, 2 oder 3 Gew.-% liegen. Nach einer bevorzugten Ausführungsform enthält das erfindungsgemäße Mittel kationische Tenside, vorzugsweise in Mengen von zumindest 0,1 Gew.-%, bezogen auf das gesamte Mittel. Nach einer anderen bevorzugten Ausführungsform kann das erfindungsgemäße Mittel frei von Kationtensid sein.

Die erfindungsgemäßen Textilwasch- oder -pflegemittel können ein oder mehrere amphotere Tenside enthalten, vorteilhafterweise in Mengen, bezogen auf die Gesamtzusammensetzung, von 0 bis 30 Gew.-%, noch vorteilhafter größer 0 bis 20 Gew.-%, vorzugsweise 0,01 bis 10 Gew.-%, insbesondere 0,1 bis 5 Gew.-%. Nach einer anderen bevorzugten Ausführungsform kann das erfindungsgemäße Mittel frei von amphoteren Tensiden sein.

Nach einer bestimmten Ausführungsform können die erfindungsgemäßen Mittel nur sehr wenig Gesamttensid enthalten, z.B. kann die Gesamttensidmenge unter 20 Gew.-%, 15 Gew.-%, 10 Gew.-% oder 5 Gew.-%, vorteilhafterweise sogar unter 3 Gew.-% oder unter 1 Gew.-%, insbesondere sogar unter 0,5 Gew.-% oder unter 0,1 Gew.-% liegen, Gew.-% jeweils bezogen auf das gesamte Mittel. Vorzugsweise beträgt der Gesamttensidgehalt aber zumindest 0,01 Gew.-%, 0,1 Gew-% oder 1 Gew.-%, bezogen auf das gesamte Mittel.

Weitere Inhaltsstoffe der erfindungsgemäßen Mittel können anorganische und organische Buildersubstanzen sein. Zu den anorganischen Buildersubstanzen gehören wasserunlösliche oder nicht wasserlösliche Inhaltsstoffe, wie Aluminosilikate und insbesondere Zeolithe.

In einer bevorzugten Ausführungsform enthält das erfindungsgemäße Mittel kein Phosphat und/oder kein Zeolith. Es ist aber auch möglich, dass das Mittel Zeolith enthält. Bevorzugt kann es dann sein, dass dieser Zeolithanteil, bezogen auf das Gesamtgewicht des Mittel, weniger als 5 Gew.-%, vorzugsweise maximal 4 Gew.-%, maximal 3 Gew.-% oder maximal 2 Gew.-% beträgt.

Es kann aber vorteilhafterweise auch vorgesehen sein, dass das erfindungsgemäße Mittel einen Zeolithgehalt von mindestens 10 Gew.-%, z.B. mindestens 15 Gew.-% oder mindestens 20 Gew.-% oder mindestens 30 Gew.-% oder auch darüber hinaus, beispielsweise mindestens 50 Gew.-% aufweist.

Lösliche Builder kann das erfindungsgemäße Mittel vorzugsweise in Mengen von 0,1 Gew.-% bis 30 Gew.-%, bevorzugt 5 Gew.-% bis 25 Gew.-% und besonders bevorzugt 10 Gew.-% bis 20 Gew.-%, bezogen auf das Gesamtgewicht des Mittel, enthalten, wobei Natriumcarbonat als löslicher Builder besonders bevorzugt ist. Es kann aber vorteilhafterweise auch vorgesehen sein, dass das erfindungsgemäße Mittel weniger als 10 Gew.-%, beispielsweise weniger als 5 Gew.-% lösliche Builder enthält. Geeignet sind beispielsweise Citrate, SKS-6, Citronensäure, MGDA (methyl glycine di-acetic acid), Triphsophate, Phosphonate, aliphatische Dicarbonsäuren (z.B. Adipin-, Glutar-, Bernsteinsäure). Nach einer anderen bevorzugten Ausführungsform kann das erfindungsgemäße Mittel frei von löslichem Builder sein.

In einer bevorzugten Ausführungsform der Erfindung sollen alle enthaltenen anorganischen Bestandteile vorzugsweise wasserlöslich sein. In diesen Ausführungsformen werden deshalb andere Buildersubstanzen als die genannten Zeolithe eingesetzt.

Eine weitere Substanzklasse mit Cobuildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z.B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

In Fällen, in denen ein Phosphat-Gehalt toleriert wird, können auch Phosphate mitverwendet werden, insbesondere Pentanatriumtriphosphat, gegebenenfalls auch Pyrophosphate sowie Orthophosphate, die in erster Linie als Fällungsmittel für Kalksalze wirken. Phosphate werden überwiegend in maschinellen Geschirrspülmitteln, teilweise aber auch noch in Waschmitteln eingesetzt

In einer bevorzugten Ausführungsform der Erfindung werden als anorganischen Buildersubstanzen insbesondere Carbonate und Silicate eingesetzt.

Zu den bevorzugten Buildersubstanzen gehören auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, welche Sekundärwascheigenschaften aufweisen. Im Rahmen dieser Erfindung wird unter dem Begriff "amorph" auch "röntgenamorph" verstanden. Dies heißt, dass die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharfe Beugungsmaxima liefern. Dies ist so zu interpretieren, dass die Produkte mikrokristalline Bereiche der Größe 10 bis einige Hundert nm aufweisen, wobei Werte bis max. 50 nm und insbesondere bis max. 20 nm bevorzugt sind. Derartige sogenannte röntgenamorphe Silikate, welche ebenfalls eine Löseverzögerung gegenüber den herkömmlichen Wassergläsern aufweisen, sind bekannt. Insbesondere bevorzugt sind verdichtete/kompaktierte amorphe Silikate, compoundierte amorphe Silikate und übertrocknete röntgenamorphe Silikate. Der Gehalt der (röntgen-)amorphen Silicate in insbesondere zeolithfreien Mitteln beträgt vorzugsweise 1 bis 10 Gew.-%, was einer bevorzugten Ausführungsform der Erfindung entspricht.

Besonders bevorzugte anorganische wasserlösliche Builder sind Alkalimetallcarbonate und Alkalimetallbicarbonate, wobei Natrium- und Kaliumcarbonat und insbesondere Natriumcarbonat zu den bevorzugten Ausführungsformen zählen. Der Gehalt der Alkalimetallcarbonate in insbesondere zeolithfreien Mitteln kann in einem sehr breiten Rahmen variieren und beträgt vorzugsweise 1 bis 50 Gew.-%, vorteilhafterweise 5 bis 40 Gew.-%, insbesondere 8 bis 30 Gew.-%, wobei üblicherweise der Gehalt an Alkalimetallcarbonaten höher ist als an (röntgen-)amorphen Silicaten. Nach einer anderen bevorzugten Ausführungsform kann das erfindungsgemäße Mittel frei von Alkalimetallcarbonaten sein.

Brauchbare organische Gerüstsubstanzen sind beispielsweise die in Form ihrer Alkali- und insbesondere Natriumsalze einsetzbaren Polycarbonsäuren, wie Citronensäure, Adipinsäure, Bernsteinsäure, Glutarsäure, Weinsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer Builderwirkung typischerweise auch die Eigenschaft einer Säuerungskomponente und dienen somit, wie beispielsweise in den erfindungsgemäßen Granulaten, auch zur Einstellung eines niedrigeren und milderen pH-Wertes von Wasch-, Pflege- und Reinigungsmitteln. Insbesondere sind hierbei Citronensäure, Bernsteinsäure, Glutarsäure, Adipinsäure, Gluconsäure und beliebige Mischungen aus diesen zu nennen.

Als organische Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol. Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Die erfindungsgemäßen Mittel können auch Polymere enthalten, insbesondere als Träger für die Parfümöle. Geeignete Polymere, die auch als Trägerstoffe in Verbindung mit Duftstoff einsetzbar sind, umfassen insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2000 bis 70000 g/mol, vorzugsweise 20000 bis 50000 g/mol und insbesondere 30000 bis 40000 g/mol.

Der Gehalt der Mittel an organischen Buildersubstanzen kann in einem breiten Rahmen variieren. Bevorzugt sind Gehalte von 2 bis 20 Gew.-%, wobei insbesondere Gehalte von maximal 10 Gew.-% besonderen Anklang finden. Nach einer anderen bevorzugten Ausführungsform kann das erfindungsgemäße Mittel frei von organischen Buildersubstanzen sein.

Es sei an dieser Stelle darauf hingewiesen, dass sich die Angabe Gew.-%, sofern es nicht anders angegeben ist, jeweils auf das gesamte Mittel bezieht.

Die erfindungsgemäßen Mittel können Komponenten aus den Klassen der Vergrauungsinhibitoren (Schmutzträger), der Neutralsalze und/oder der textilweichmachenden Hilfsmittel (beispielsweise Kationtenside) aufweisen, was bevorzugt ist.

Als typisches Beispiel für einen geeigneten Vertreter der Neutralsalze ist das Natriumsulfat zu nennen. Es kann in Mengen von beispielsweise 0 bis 60 Gew.-%, vorzugsweise 2 bis 45 Gew.-% eingesetzt werden.

Geeignete Weichmacher sind beispielsweise quellfähige Schichtsilikate von der Art entsprechender Montmorillonite, beispielsweise Bentonit, ebenso kationische Tenside.

Der Gehalt an Wasser im Mittel richtet sich u.a. danach, ob das Mittel in flüssiger oder fester Form vorliegt, beträgt daher vorzugsweise 0 bis weniger als 100 Gew.-% und insbesondere 0,5 bis 95 Gew.-%, wobei Werte von maximal 5 Gew.-% insbesondere bei festen oder nichtwäßrigen flüssigen Mitteln besondere Bevorzugung finden. Nicht miteingerechnet wurde hierbei im Falle der festen Mittel das an gegebenenfalls vorhandene Aluminosilikate wie Zeolith anhaftende Wasser.

Im Falle flüssiger Mittel enthält das erfindungsgemäße Mittel nach einer bevorzugten Ausführungsform Wasser in einer Menge von mehr als 20 Gew.-%, vorteilhafterweise mehr als 30 Gew.%., in weiter vorteilhafter Weise mehr als 40 Gew.-%, noch vorteilhafter mehr als 50 Gew.-%, insbesondere 60 bis 99 Gew.-%, besonders bevorzugt 70 bis 98 Gew.-% und äußerst bevorzugt 80 bis 95 Gew.-%, bezogen auf das gesamte Mittel.

Die Obergrenze an Wasser kann auch bei 80 Gew.-%, 70 Gew.-%, 60 Gew.-%, 50 Gew.-%, 40 Gew.-%, 30 Gew.-%, 20 Gew.-% oder 10 Gew.-% Gew.-% liegen, bezogen auf das gesamte Mittel.

Die Untergrenze an Wasser kann z.B. auch bei 80 Gew.-%, 70 Gew.-%, 60 Gew.-%, 50 Gew.-%, 40 Gew.-%, 30 Gew.-%, 20 Gew.-% oder 10 Gew.-% liegen, bezogen auf das gesamte Mittel.

Die genannten Ober- und Untergrenzen können natürlich sinnvoll kombiniert werden, z.B. zu Wassergehalten von 60-80 Gew.-% oder 10-30 Gew.-% usw.

Das erfindungsgemäße Mittel kann, so es ein Feststoff ist, ein hervorragendes Rieselverhalten aufweisen. Nach einer bevorzugten Ausführungsform ist das erfindungsgemäße Mittel im wesentlichen festförmig, liegt vorzugsweise in pulvriger, gepresster oder granulärer Form vor.

Die erfindungsgemäßen Mittel können vorzugsweise mit weiteren Bestandteilen, insbesondere von Wasch-, Pflege-, und/oder Reinigungsmitteln oder kosmetischen Inhaltsstoffen, vermischt werden. Aus dem breiten Stand der Technik ist allgemein bekannt, welche Inhaltsstoffe von Wasch-, Pflege- oder Reinigungsmitteln und welche Rohstoffe üblicherweise noch zugemischt werden können. Es handelt sich hierbei beispielsweise um Stoffe wie Bleichmittel, Bleichaktivatoren und/oder Bleichkatalysatoren, Enzyme, temperaturempfindliche Farbstoffe usw., welche natürlich auch direkt im Mittel enthalten sein können.

Vorzugsweise können die erfindungsgemäßen Mittel, so sie denn fest sind, auch als Tablette oder Formkörper vorliegen. Als "Tablette" oder "Formkörper" werden im Rahmen der vorliegenden Anmeldung unabhängig von der Art ihrer Herstellung formstabile, feste Körper bezeichnet. Derartige Körper lassen sich beispielsweise durch Kristallisation, Formguß, Spritzguß, reaktive oder thermische Sinterung, (Co)Extrusion, Verprillung, Pastillierung, oder Kompaktierungsverfahren wie die Kalandrierung oder Tablettierung herstellen. Die Herstellung der "Tabletten" oder "Formkörper" durch Tablettierung ist im Rahmen der vorliegenden Anmeldung besonders bevorzugt. Die Tablette besteht also vorzugsweise aus verpresstem, teilchenförmigen Material.

Weiterhin können die erfindungsgemäßen Mittel in Form eines Konditioniermittels und/oder Konditioniersubstrats vorliegen und die gemäßen Komponenten enthalten. Unter dem Begriff Konditionierung ist im Sinne dieser Erfindung vorzugsweise die avivierende Behandlung von Textilien, Stoffen und Geweben zu verstehen. Durch die Konditionierung werden den Textilien positive Eigenschaften verliehen, wie beispielsweise ein verbesserter Weichgriff, eine erhöhte Glanz- und Farbbrillanz, ein verbesserter Dufteindruck, Verringerung der Filzbildung, Bügelerleichterung durch Verringerung der Gleiteigenschaften, Verringerung des Knitterverhaltens und der statischen Aufladung sowie eine Farbübertragungsinhibierung bei gefärbten Textilien.

Erfindungsgemäße Konditioniermittel können vorteilhafterweise einen pH Wert von kleiner oder gleich 8, vorzugsweise kleiner 7, besonders bevorzugt zwischen 1 und 6 und insbesondere zwischen 2 und 5 aufweisen.

Die erfindungsgemäßen Konditioniermittel können in einer bevorzugten Ausführungsform zusätzlich Tenside enthalten. Der zusätzliche Einsatz von Tensiden bewirkt eine Verstärkung der konditionierenden Eigenschaften und trägt zudem zu einer verbesserten Lagerstabilität und Dispergierbarkeit beziehungsweise Emulgierbarkeit der einzelnen Konditionierungsmittelkomponenten bei.

Zur Verbesserung des Weichgriffs und der avivierenden Eigenschaften können die erfindungsgemäßen Mittel Weichmacherkomponenten aufweisen. Beispiele für solche Verbindungen sind quartäre Ammoniumverbindungen, kationische Polymere und Emulgatoren, wie sie in Haarpflegemitteln und auch in Mitteln zur Textilavivage eingesetzt werden. Diese weichmachenden Verbindungen, welche auch nachfolgend näher beschreiben werden, können in allen erfindungsgemäßen Mitteln, insbesondere aber in den Konditioniermitteln bzw. in Mitteln mit angestrebter weichmachender Wirkung, enthalten sein.

Besonders bevorzugt sind alkylierte quaternäre Ammoniumverbindungen, von denen mindestens eine Alkylkette durch eine Estergruppe und/oder Amidogruppe unterbrochen ist, insbesondere N-Methyl-N(2-hydroxyethyl)-N,N-(ditalgacyloxyethyl)ammonium-methosulfat.

In einem erfindungsgemäßen Mittel, vorzugsweise Konditionierungsmittel können Weichmacher in Mengen von 0,1 bis 80 Gew.-%, üblicherweise 0,1 bis 70 Gew.-%, vorzugsweise 0,2 bis 60 Gew.-% und insbesondere 0,5 bis 40 Gew.-%, jeweils bezogen auf das gesamte Mittel, enthalten sein.

In einer bevorzugten Ausführungsform liegen die erfindungsgemäßen Mittel in flüssiger Form vor, beispielsweise in Form von Konditioniermittel oder Flüssigwaschmittel usw. Zum Erreichen einer flüssigen Konsistenz kann der Einsatz sowohl flüssiger organischer Lösungsmittel, wie auch der von Wasser angezeigt sein. Die erfindungsgemäßen Mittel enthalten daher gegebenenfalls Lösungsmittel.

Lösungsmittel, die in den erfindungsgemäßen Mitteln eingesetzt werden können, stammen beispielsweise aus der Gruppe ein- oder mehrwertigen Alkohole, Alkanolamine oder Glycolether, so-fern sie im angegebenen Konzentrationsbereich mit Wasser mischbar sind. Vorzugsweise werden die Lösungsmittel ausgewählt aus Ethanol, n- oder i-Propanol, Butanolen, Glykol, Propan- oder Butandiol, Glycerin, Diglykol, Propyl- oder Butyldiglykol, Hexylenglycol, Ethylenglykolmethylether, Ethylenglykolethylether, Ethylen-glykolpropylether, Ethylenglykolmono-n-butylether, Diethylenglykol-methylether, Diethylenglykolethylether, Propylenglykolmethyl-, -ethyl- oder -propyl-ether, Butoxy-propoxy-propanol (BPP), Dipropylenglykolmonomethyl-, oder -ethylether, Di-isopropylenglykolmonomethyl-, oder -ethylether, Methoxy-, Ethoxy- oder Butoxytriglykol, 1-Butoxyethoxy-2-propanol, 3-Methyl-3-methoxybutanol, Propylen-glykol-t-butylether sowie Mischungen dieser Lösungsmittel.

Das erfindungsgemäße Mittel, vorzugsweise Konditioniermittel oder Flüssigwaschmittel, kann ein oder mehrere Lösungsmittel in einer Menge von üblicherweise bis zu 90 Gew.-%, vorzugsweise 0,1 bis 30 Gew.-%, insbesondere 2 bis 20 Gew.-%, besonders bevorzugt 3 bis 15 Gew.-%, äußerst bevorzugt 5 bis 12 Gew.-%, beispielsweise 5,3 oder 10,6 Gew.-%, jeweils bezogen auf das gesamte Mittel, enthalten.

Das erfindungsgemäße Textilwasch- oder -pflegemittel, wie insbesondere Konditioniermittel, enthält vorteilhafterweise Komplexbildner in einer Menge von üblicherweise 0 bis 20 Gew.-%, vorzugsweise 0,1 bis 15 Gew.-%, insbesondere 0,5 bis 10 Gew.-%, besonders bevorzugt 1 bis 8 Gew.-%, äußerst bevorzugt 1,5 bis 6 Gew.-%, bezogen auf das gesamte Mittel.

In einer weiteren Ausführungsform enthält das erfindungsgemäße Textilwasch- oder -pflegemittel, wie insbesondere Konditioniermittel, gegebenenfalls ein oder mehrere Viskositätsregulatoren, die vorzugsweise als Verdicker fungieren.

Die Viskosität von erfindungsgemäßen Mitteln, welche flüssig sind, kann mit üblichen Standardmethoden (beispielsweise Brookfield-Viskosimeter RVD-VII bei 20 U/min und 20°C, Spindel 3) gemessen werden und liegt vorzugsweise im Bereich von 10 bis 5000 mPas. Bevorzugte flüssige bis gelförmige Mittel haben Viskositäten von 20 bis 4000 mPas, wobei Werte zwischen 40 und 2000 mPas besonders bevorzugt sind.

Geeignete Verdicker sind anorganische oder polymere organische Verbindungen. Es können auch Gemische aus mehreren Verdickern eingesetzt werden.

Zu den anorganischen Verdickern zählen beispielsweise Polykieselsäuren, Tonmineralien wie Montmorillonite, Zeolithe, Kieselsäuern, Aluminiumsilikate, Schichtsilikate und Bentonite.

Die organischen Verdicker stammen aus den Gruppen der natürlichen Polymere, der abgewandelten natürlichen Polymere und der vollsynthetischen Polymere.

In einer weiteren bevorzugten Ausführungsform enthält das erfindungsgemäße Textilwasch- oder -pflegemittel, wie insbesondere Konditioniermittel, gegebenenfalls ein oder mehrere Enzyme.

Als Enzyme kommen insbesondere solche aus der Klassen der Hydrolasen wie der Proteasen, Esterasen, Lipasen bzw. lipolytisch wirkende Enzyme, Amylasen, Cellulasen bzw. andere Glykosylhydrolasen und Gemische der genannten Enzyme in Frage. Alle diese Hydrolasen tragen in der Wäsche zur Entfernung von Verfleckungen wie protein-, fett- oder stärkehaltigen Verfleckungen und Vergrauungen bei. Cellulasen und andere Glykosylhydrolasen können darüber hinaus durch das Entfernen von Pilling und Mikrofibrillen zur Farberhaltung und zur Erhöhung der Weichheit des Textils beitragen. Zur Bleiche bzw. zur Hemmung der Farbübertragung können auch Oxireduktasen eingesetzt werden.

Die Enzyme können als Formkörper an Trägerstoffe adsorbiert oder gecoated eingebettet sein, um sie gegen vorzeitige Zersetzung zu schützen. Der Anteil der Enzyme, Enzymmischungen oder Enzymgranulate kann beispielsweise etwa 0,1 bis 5 Gew.-%, vorzugsweise 0,12 bis etwa 2 Gew.-% betragen, bezogen auf das gesamte Mittel.

Die erfindungsgemäßen Textilwasch- oder -pflegemittel (z.B. Konditioniermittel), können gegebenenfalls Bleichmittel enthalten. Unter den als Bleich-mittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxopyrophosphate, Citratperhydrate sowie H₂O₂ liefernde persaure Salze oder Persäuren, wie Persulfate beziehungsweise Perschwefelsäure. Brauchbar ist auch das Harnstoffperoxohydrat Percarbamid, das durch die Formel H₂N-CO-NH₂·H₂O₂ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind die Peroxybenzoesäure und ihre ringsubstituierten Derivate, wie Alkylperoxybenzoesäuren, aber auch Peroxy-α-Naphtoesäure und Magnesium-monoperphthalat, die aliphatischen oder substituiert aliphatischen Peroxysäuren, wie Peroxylaurinsäure, Peroxystearinsäure, ε-Phthalimidoperoxycapronsäure (Phthalimidoperoxyhexansäure, PAP), o-Carboxybenzamidoperoxycapronsäure, N-Nonenylamidoperadipinsäure und N-Nonenylamidopersuccinate, und aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyl-diperoxybutan-1,4-di-säure, N,N-Terephthaloyl-di(6-aminopercapronsäure) können eingesetzt werden.

Weiterhin können die erfindungsgemäßen Textilwasch- oder -pflegemittel gegebenenfalls Bügelhilfsstoffe zur Verbesserung des Wasserabsorptionsvermögens, der Wiederbenetzbarkeit der behandelten Textilien und zur Erleichterung des Bügelns der behandelten Textilien enthalten. Es können in den Formulierungen beispielsweise Silikonderivate eingesetzt werden. Diese verbessern zusätzlich das Ausspülverhalten der waschaktiven Formulierungen durch ihre schauminhibierenden Eigenschaften. Bevorzugte Silikonderivate sind beispielsweise Polydialkyl- oder Alkylarylsiloxane, bei denen die Alkylgruppen ein bis fünf C-Atome aufweisen und ganz oder teilweise fluoriert sind. Bevorzugte Silikone sind Polydimethylsiloxane, die gegebenenfalls derivatisiert sein können und dann aminofunktionell oder quaterniert sind bzw. Si-OH-, Si-H- und/oder Si-Cl-Bindungen aufweisen. Die Viskositäten der bevorzugten Silikone liegen bei 25°C im Bereich zwischen 100 und 100.000 mPas, wobei die Silikone in Mengen zwischen 0,2 und 5 Gew.-%, bezogen auf das gesamte Mittel eingesetzt werden können.

Die erfindungsgemäßen Mittel, insbesondere Konditioniermittel, können nach allen bekannten, dem Fachmann geläufigen Techniken erhalten werden. Die Mittel können beispielsweise durch Aufmischen unmittelbar aus ihren Rohstoffen, gegebenenfalls unter Einsatz von hochscherenden Mischapparaturen erhalten werden. Für flüssige Formulierungen, insbesondere Konditioniermittel, empfiehlt sich z.B. ein Aufschmelzen, gegebenenfalls vorhandener Weichmacherkomponenten und ein nachfolgendes Dispergieren der Schmelze in einem Lösungsmittel, vorzugsweise Wasser.

Vorzugsweise liegen die Konditioniermittel als Weichspülmittel vor. Sie werden dabei üblicherweise in den Nachspülgang einer automatischen Waschmaschine eingebracht.

Erfindungsgemäße Konditioniersubstrate finden ihren Einsatz vor allem in der Textilbehandlung und insbesondere in Textiltrocknungsverfahren. Das Substratmaterial besteht vorzugsweise aus porösen flächigen Tüchern. Sie können aus einem faserigen oder zellulären flexiblen Material bestehen, das ausreichend thermische Stabilität zur Verwendung im Trockner aufweist und das ausreichende Mengen eines Imprägnierungs- bzw. Beschichtungsmittels zurückhalten kann, um Stoffe effektiv zu konditionieren, ohne dass während der Lagerung ein nennenswertes Auslaufen oder Ausbluten des Mittels erfolgt. Zu diesen Tüchern gehören Tücher aus gewebtem und ungewebtem synthetischen und natürlichen Fasern, Filz, Papier oder Schaumstoff, wie hydrophilem Polyurethanschaum.

Ein weiterer Gegenstand der Erfindung ist die Verwendung eines erfindungsgemäßen Konditioniermittels oder eines erfindungsgemäßen Konditioniersubstrats in einem Textilkonditionierverfahren, wie beispielsweise einem Nachspülgang, einem Textiltrocknungsverfahren und einem Textiltrockenreinigungs- oder Textilauffrischungsverfahren. Ebenso ist ein konditioniertes Substrat, z.B. aus Holz, Papier, Leder o.ä. als Raumbeduftungsmittel einsetzbar.

Ein erfindungsgemäßes, pulverförmiges Vollwaschmittel kann neben dem lichtaktiven Bleichmittel vorzugsweise z.B. Komponenten enthalten, die ausgewählt sind aus den folgenden:
- Aniontenside, wie z.B. Alkylbenzolsulfonat, Alkylsulfat , in Mengen von vorteilhafterweise 5-30 Gew.-%, vorzugsweise 8-15 Gew.-%, insbesondere 15-20 Gew.-%,
- Nichtionische Tenside, wie z.B. Fettalkoholpolyglycolether, Alkylpolyglucosid, Fettsäureglucamid, vorteilhafterweise 0,1-20 Gew.-%, vorzugsweise 2-15 Gew.-%, insbesondere 6-11 Gew.-%
- Gerüststoffe, wie z.B. Zeolith, Polycarboxylat, Natriumcitrat, 5-60 Gew.-%, vorzugsweise 10-55 Gew.-%, insbesondere 15-40 Gew.-%,
- Alkalien, wie z.B. Natriumcarbonat, vorteilhafterweise 1-30 Gew.-%, vorzugsweise 2-25 Gew.-%, insbesondere 5-20 Gew.-%,
- zusätzliche Bleichmittel, wie z.B. Natriumperborat, Natriumpercarbonat, vorteilhafterweise 5-25 Gew.-%, vorzugsweise 10-20 Gew.-%,
- Korrosionsinhibitoren, z.B. Natriumsilicat, vorteilhafterweise 1-6 Gew.-%, vorzugsweise 2-5 Gew.-%, insbesondere 3-4 Gew.-%,
- Stabilisatoren, z.B. Phosphonate vorteilhafterweise 0-1 Gew.-%,
- Schauminhibitor, z.B. Seife, Siliconöle, Paraffine vorteilhafterweise 0,1-4 Gew.-%, vorzugsweise 0,2-2 Gew.-%, insbesondere 1-3 Gew.-%,
- Enzyme, z.B. Proteasen, Amylasen, Cellulasen, Lipasen, vorteilhafterweise 0,1-2 Gew.-%, vorzugsweise 0,2-1 Gew.-%, insbesondere 0,3-0,8 Gew.-%,
- Vergrauungsinhibitor, z.B. Carboxymethylcellulose, vorteilhafterweise 0-1 Gew.-%,
- Verfärbungsinhibitor, z.B. Polyvinylpyrrolidon-Derivate, vorteilhafterweise 0-2 Gew.-%,
- Stellmittel, z.B. Natriumsulfat, vorteilhafterweise 0-20 Gew.-%,
- Optische Aufheller, z.B. Stilben-Derivat, Biphenyl-Derivat, vorteilhafterweise 0,1-0,4 Gew.-%, insbesondere 0,1-0,3 Gew.-%,
- Duftstoffe
- Wasser
- Seife
- Bleichaktivatoren
- Cellulosderivate
- Schmutzabweiser.

Ein erfindungsgemäßes flüssiges Vollwaschmittel kann neben dem lichtaktiven Bleichmittel vorzugsweise z.B. folgende Komponenten enthalten, die ausgewählt sind aus den folgenden:
- Aniontenside, wie z.B. Alkylbenzolsulfonat, Alkylsulfat , in Mengen von vorteilhafterweise 5-40 Gew.-%, vorzugsweise 8-30 Gew.-%, insbesondere 15-25 Gew.-%,
- Nichtionische Tenside, wie z.B. Fettalkoholpolyglycolether, Alkylpolyglucosid, Fettsäureglucamid, vorteilhafterweise 0,1-25 Gew.-%, vorzugsweise 5-20 Gew.-%, insbesondere 10-15 Gew.-%,
- Gerüststoff, wie z.B. Zeolith, Polycarboxylat, Natriumcitrat, vorteilhafterweise 0-15 Gew.-%, vorzugsweise 0,01-10 Gew.-%, insbesondere 0,1-5 Gew.-%,
- Schauminhibitor, z.B. Seife, Siliconöle, Paraffine, vorteilhafterweise 0,1-.4 Gew.-%, vorzugsweise 0,2-3 Gew.-%, insbesondere 1-2 Gew.-%,
- Enzyme, z.B. Proteasen, Amylasen, Cellulasen, Lipasen, vorteilhafterweise 0,1-2 Gew.-%, vorzugsweise 0,2-1 Gew.-%, insbesondere 0,3-0,8 Gew.-%,
- Optische Aufheller, z.B. Stilben-Derivat, Biphenyl-Derivat, vorteilhafterweise 0,1-0,4 Gew.-%, insbesondere 0,1-0,3 Gew.-%,
- Duftstoffe
- -Stabilisatoren,
- Wasser
- Seife, vorteilhafterweise 1-20 Gew.-%, vorzugsweise 2-15 Gew.-%, insbesondere 5-10 Gew.-%,
- Alkohole/Lösungsmittel, vorteilhafterweise 0-25 Gew.-%, vorzugsweise 1-20 Gew.-%" insbesondere 2-15 Gew.-%,

Die erfindungsgemäßen Mittel können vorzugsweise auch mit Parfümöl (Riechstoffe, Duftstoffe) parfümiert sein.

Mit dem Begriff Parfümöl sind vorzugsweise in sich abgeschlossene Duftstoffkompositionen gemeint, welche gemeinhin zur Produktbeduftung eingesetzt werden und insbesondere nach menschlichem Ermessen wohlriechend sind. Dies sei an einem Beispiel erläutert. Will ein Fachmann z.B. ein Duschgel wohlriechend machen, so fügt er ihm für gewöhnlich nicht nur eine (wohl-) riechende Substanz, sondern ein Kollektiv (wohl-)riechender Substanzen bei. Ein solches Kollektiv besteht gewöhnlich aus einer Vielzahl einzelner Riechstoffe, z. B. mehr als 10 oder 15, vorzugsweise bis zu 100 oder mehr. Diese Reichstoffe formen zusammenwirkend ein gewünschtes wohlriechendes, harmonisches Geruchsbild.

Ein erfindungsgemäßes Parfümöl kann einzelne Riechstoffverbindungen, z.B. die synthetischen Produkte vom Typ der Ester, Ether, Aldehyde, Ketone, Alkohole und Kohlenwasserstoffe enthalten. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexyl-propionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, ∝-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote des gebildeten Parfümöl erzeugen.

Die Parfümöle können aber auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Um wahrnehmbar zu sein, muß ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms während des Verdampfens, wobei man die Geruchseindrücke in "Kopfnote" (top note), "Herz- bzw. Mittelnote" (middle note bzw. body) sowie "Basisnote" (end note bzw. dry out) unterteilt.

Haftfeste Riechstoffe, die im Rahmen der vorliegenden Erfindung vorteilhafterweise in den Parfümölen einsetzbar sind, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lemongrasöl, Moschuskömeröl, Myrrhenöl, Nelkenöl, Neroliöl, Niaouliöl, Olibanumöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Sternanisöl, Thujaöl, Thymianöl, Verbenaöl, Vetiveröl, Wacholderbeeröl, Wermutöl, Wintergrünöl, Ylang -Ylang-Öl, Y-sop-Öl, Zimtöl, Zimtblätteröl sowie Zypressenöl.

Aber auch die höhersiedenden bzw. festen Riechstoffe natürlichen oder synthetischen Ursprungs können im Rahmen der vorliegenden Erfindung vorteilhafterweise als haftfeste Riechstoffe bzw. Riechstoffgemische in den Parfümölen eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Di-methylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p- Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, Nitrobenzol, n-Nonylaldehyd, Nonylakohol, n-Octylaldehyd, p-Oxy-Acetophenon, Pentadekanolid, β-Phenylethylakohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimtalkohol, Zimtsäure, Zimtsäureethylester, Zimtsäurebenzylester.

Zu den leichter flüchtigen Riechstoffen, die im Rahmen der vorliegenden Erfindung in den Parfümöl vorteilhaft einsetzbar sind, zählen insbesondere die niedriger siedenden Riechstoffe natürlichen oder synthetischen Usprung, die allein oder in Mischungen eingesetzt werrden können. Beispiele für leichter flüchtige Riechstoffe sind Alkyisothiocyanate (Alkylsenföle), Butandion, Limonen, Linalool, Linaylacetat und -propionat, Menthol, Menthon, Methyl-n-hep-tenon, Phellandren, Phenylacetaldehyd, Terpinylacetat, Zitral, Zitronellal.

Alle vorgenannten Riechstoffe sind alleine oder in Mischung in den Parfümölen gemäß der vorliegenden Erfindung mit den bereits genannten Vorteilen einsetzbar.

In einer bevorzugten Ausführungsform enthält das erfindungsgemäße Mittel bestimmte Minimalwerte an Parfümöl, nämlich zumindest 0,00001 Gew.-%, vorteilhafterweise zumindest 0,0001 Gew.-%, in beträchtlich vorteilhafter Weise zumindest 0,001 Gew.-%, in vorteilhafterer Weise zumindest 0,01 Gew.-%, in weiter vorteilhafter Weise zumindest 0,1 Gew.-%, in noch weiter vorteilhafter Weise zumindest 0,2 Gew.-%, in sehr vorteilhafter Weise zumindest 0,3 Gew.-%, in besonders vorteilhafter Weise zumindest 0,4 Gew.-%, in ganz besonders vorteilhafter Weise zumindest 0,45 Gew.-%, in erheblich vorteilhafter Weise zumindest 0,5 Gew.-%, in ganz erheblich vorteilhafter Weise zumindest 0,55 Gew.-%, in äußerst vorteilhafter Weise zumindest 0,6 Gew.-%, in höchst vorteilhafterweise zumindest 0,65 Gew.-%, in überaus vorteilhafterweise zumindest 0,7 Gew.-%, in ausnehmend vorteilhafter Weise zumindest 0,75 Gew.-%, in außergewöhnlich vorteilhafter Weise zumindest 0,8 Gew.-%, in außerordentlich vorteilhafter Weise zumindest 0,85 Gew.-%, insbesondere zumindest 0,9 Gew.-% an Parfümöl, bezogen auf das gesamte Mittel.

In einer bevorzugten Ausführungsform enthalten die Parfümöle weniger als 8 , vorteilhafterweise weniger als 7, in vorteilhafterer Weise weniger als 6, in wiederum vorteilhafterer Weise weniger als 5, in weiter vorteilhafterweise weniger als 4, noch vorteilhafter weniger als 3, vorzugsweise weniger als 2, insbesondere keine Duftstoffe aus der Liste Amylcinnamal, Amylcinnamylalkohol, Benzylalkohol, Benzylsalicylat, Cinnamylalkohol, Cinnamal, Citral, Cumarin, Eugenol, Geraniol, Hydroxycitronellal, Hydroxymethylpentylcyclohexencarboxaldehyd, Isoeugenol, Anisylalkohol, Benzylbenzoat, Benzylcinnamat, Citronellol, Farnesol, Hexylcinnamaldehyd, Lilial, d-Limonen, Linalool, Methylheptincarbonat, 3-Methyl-4-(2,6,6-trimethyl-2-cyclohexen-1-yl)-3-buten-2-on, Eichenmoosextrakt, Baummoosextrakt.

Nach einer weiteren bevorzugten Ausführungsform kann das erfindungsgemäße Mittel frei von Parfümöl sein.

## Patentansprüche

1. Textilwasch- oder -pflegemittel, welches 0,01 bis 25 Gew.-% mit Kohlenstoff modifiziertes Titandioxid, bezogen auf das gesamte Mittel, als lichtaktives Bleichmittel umfasst, wobei das Mittel in einer lichtundurchlässigen Verpackung enthalten ist

2. Mittel nach Anspruch 1, **dadurch gekennzeichnet, dass** das mit Kohlenstoff modifizierte Titandioxid in Mengen von 0,01 bis 5 Gew.-% enthalten ist, bezogen auf das gesamte Mittel.

3. Mittel nach einem der Ansprüche 1 oder 2, **dadurch gekennzeichnet, dass** der Kohlenstoffgehalt des mit Kohlenstoff modifizierten Titandioxids im Bereich von 0,01 bis 10 Gew.-% vorzugsweise von 0,05 bis 5,0 Gew.-%, vorteilhafterweise von 0,3 bis 1,5 Gew.% , insbesondere von 0,4 bis 0,8 Gew.% liegt.

4. Mittel nach einem der Ansprüche 1 bis 3, **dadurch gekennzeichnet, dass** die spezifische Oberfläche des modifizierten Titandioxids nach BET vorzugsweise 50 bis 500 m²/g, vorteilhafterweise 100 bis 400 m²/g, insbesondere 200 bis 350 m²/g beträgt.

5. Mittel nach einem der Ansprüche 1 bis 4, **dadurch gekennzeichnet, dass** die Teilchengröße des modifizierten Titandioxid im Bereich von 2-600 nm, vorzugsweise im Bereich von 200-400 nm liegt.

6. Mittel nach einem der Ansprüche 1 bis 5, **dadurch gekennzeichnet, dass** es neben dem modifizierten Titandioxid wenigstens einen weiteren, vorzugsweise mehrere Inhaltsstoffe aufweist, insbesondere wasch-, pflege- und/oder reinigungsaktive Inhaltsstoffe, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositionsmittel, Antistatika, Buildersubstanzen, Bleichmittel, Bleichaktivatoren, Bleichstabilisatoren, Bieichkatalysatoren, Bügelhilfsmiltel, Cobuilder, Dispergiermittel, Einlaufverhinderer, Elektrolyte, Enzyme, Farbschutzstoffe, Färbemittel, Farbstoffe, Farbübertragungsinhibitoren, Fluoreszensmittel, Fungizide, Germizide, geruchskomplexierende Substanzen, Hilfsmittel, Hydrotrope, Klarspüler, Komplexbildner, Konservierungsmittel, Korrosionsinhibitoren, optische Aufheller, Parfümträger, Perlglanzgeber, pH-Stellmittel, Phobier- und Imprägniermittel, Polymere, Riechstoffe, Quell- und Schiebefestmittel, Schauminhibitoren, Schichtsilikate, schmutzabweisende Stoffe, Silberschutzmittel, Silikonöle, UV-Schutz-Substanzen, Viskositätsregulatoren, Verdickungs-mittel, Verfärbungsinhibitoren, Vergrauungsinhibitoren, Vitamine und/oder Weichspüler.

7. Verfahren zur Textilbehandlung, umfassend die Kontaktierung des Textils mit einem Textilwasch-oder -pflegemittel nach einem der Ansprüche 1-6, bei und/oder gefolgt von einer Exponierung der Oberfläche des Textils an Licht mit einer Wellenlänge im Bereich von 300-1200 nm.

## Claims

1. A textile washing or care-providing agent that comprises as a light-active bleaching agent 0.01 to 25 wt% titanium dioxide modified with carbon, based on the entire agent, the agent being contained in an opaque package.

2. The agent according to Claim 1, wherein the titanium dioxide modified with carbon is contained in quantities from 0.01 to 5 wt%, based on the entire agent.

3. The agent according to one of Claims 1 or 2, wherein the carbon content of the titanium dioxide modified with carbon is in the range from 0.01 to 10 wt%, by preference from 0.05 to 5.0 wt%, advantageously from 0.3 to 1.5 wt%, in particular from 0.4 to 0.8 wt%.

4. The agent according to one of Claims 1 to 3, wherein the specific surface area of the modified titanium dioxide according to BET is by preference 50 to 500 m²/g, advantageously 100 to 400 m²/g, in particular 200 to 350 m²/g_{.}

5. The agent according to one of Claims 1 to 4, wherein the particle size of the modified titanium dioxide is in the range from 2 to 600 nm, by preference in the range from 200 to 400 nm.

6. The agent according to one of Claims 1 to 5, wherein it comprises, besides the modified titanium dioxide, at least one further, preferably several, ingredients, in particular ingredients having washing, care-providing, and/or cleaning activity, advantageously selected from the group comprising anionic surfactants, cationic surfactants, amphoteric surfactants, non-ionic surfactants, acidifying agents, alkalizing agents, anti-wrinkle compounds, antibacterial substances, antioxidants, antiredeposition agents, antistatic agents, builder substances, bleaching agents, bleach activators, bleach stabilizers, bleach catalysts, ironing adjuvants, cobuilders, dispersants, shrinkage preventers, electrolytes, enzymes, colour protection substances, colouring agents, dyes, colour transfer inhibitors, fluorescing agents, fungicides, germicides, odour-complexing substances, adjuvants, hydrotropes, rinse aids, complexing agents, preservatives, corrosion inhibitors, optical brighteners, perfume carriers, lustre agents, pH adjusting agents, proofing and impregnation agents, polymers, fragrances, swelling and anti-slip agents, foam inhibitors, sheet silicates, soil-repellent substances, silver protectants, silicone oils, UV protective substances, viscosity regulators, thickening agents, discoloration inhibitors, anti-grey agents, vitamins, and/or fabric softeners.

7. A method for textile treatment, comprising bringing the textile into contact with a textile washing or care-providing agent according to one of Claims 1 to 6 during and/or followed by an exposure of the surface of the textile to light having a wavelength in the range from 300 to 1200 nm.

## Revendications

1. Agent de lavage ou d'entretien des textiles, qui comprend de 0,01 à 25 % en poids de dioxyde de titane modifié avec du carbone, rapportés à la totalité de l'agent, à titre d'agent de blanchiment actif sous l'effet de la lumière, l'agent étant contenu dans un emballage opaque.

2. Agent selon la revendication 1, **caractérisé en ce que** le dioxyde de titane modifié avec du carbone est contenu dans des quantités de 0,01 à 5 % en poids, rapportés à l'agent dans sa totalité.

3. Agent selon l'une quelconque des revendications 1 ou 2, **caractérisé en ce que** la teneur en carbone du dioxyde de titane modifié avec du carbone se situe dans la plage de 0,01 à 10 % en poids, de préférence de 0,05 à 5,0 % en poids, de manière avantageuse de 0,3 à 1,5 % en poids, en particulier de 0,4 à 0,8 % en poids.

4. Agent selon l'une quelconque des revendications 1 à 3, **caractérisé en ce que** la surface spécifique du dioxyde de titane modifié selon BET s'élève de préférence de 50 à 500 m²/g, de préférence de 100 à 400 m²/g, en particulier de 200 à 350 m²/g.

5. Agent selon l'une quelconque des revendications 1 à 4, **caractérisé en ce que** la granulométrie du dioxyde de titane modifié se situe dans la plage de 2 à 600 nm, de préférence dans la plage de 200 à 400 nm.

6. Agent selon l'une quelconque des revendications 1 à 5, **caractérisé en ce qu'**il présente, à côté du dioxyde de titane modifié, au moins un constituant supplémentaire, de préférences plusieurs constituants supplémentaires, en particulier des constituants possédant une activité de lavage, d'entretien et/ou de nettoyage, qui sont choisis de manière avantageuse parmi le groupe comprenant des agents tensioactifs anioniques, des agents tensioactifs cationiques, des agents tensioactifs amphotères, des agents tensioactifs non ioniques, des agents d'acidification, des agents d'alcalinisation, des agents antifroissement, des substances antibactériennes, des antioxydants, des agents antiredéposition, des agents antistatiques, des substances faisant office de builders, des agents de blanchiment, des activateurs du blanchiment, des stabilisateurs du blanchiment, des catalyseurs du blanchiment, des agents facilitant le repassage, des cobuilders, des agents de mise en dispersion, des agents empêchant le rétrécissement, des électrolytes, des enzymes, des agents de protection des couleurs, des teintures, des colorants, des inhibiteurs du transfert des couleurs, des agents fluorescents, des fongicides, des germicides, des substances de complexation des odeurs, des adjuvants, des hydrotropes, des liquides de rinçage, des formateurs de complexes, des conservateurs, des inhibiteurs de la corrosion, des azurants optiques, des porteurs de parfums, des agents conférant un reflet nacré, des agents de réglage du pH, des agents rendant hydrophobe et des agents d'imprégnation, des polymères, des fragrances, des agents s'opposant au gonflement et au glissement, des inhibiteurs de mousse, des silicates stratifiés, des substances repoussant les saletés, des agents de protection de l'argent, des huiles de silicone, des substances de protection contre l'exposition aux rayonnements ultraviolets, des régulateurs de la viscosité, des épaississants, des inhibiteurs de la décoloration, des inhibiteurs du grisaillement, des vitamines et/ou des agents assouplissants.

7. Procédé pour le traitement des textiles, comprenant la mise en contact du textile avec un agent de lavage ou d'entretien des textiles selon l'une quelconque des revendications 1 à 6, lors d'une exposition et/ou suivie d'une exposition de la surface du textile à la lumière possédant une longueur d'ondes dans la plage de 300 à 1200 nm.
